# EUROPEAN PATENT SPECIFICATION

(11) **EP 4 223 207 B1**
(45) Date of publication and mention of the grant of the patent: **14.10.2026**
(21) Application number: 23173018.5
(22) Date of filing: 29.01.2018
(51) Int. Cl.: A61B 1/00, A61B 1/05, A61B 1/018

(54) **ENDOSCOPE HAVING MULTIPLE VIEWING DIRECTIONS**
ENDOSKOP MIT MEHREREN ANSICHTSRICHTUNGEN
ENDOSCOPE AYANT DE MULTIPLES DIRECTIONS DE VISUALISATION

(30) Priority: 01.02.2017 US 201762453186 P
(43) Date of publication of application: 09.08.2023
(62) Document number(s) of the earlier application(s) in accordance with Art. 76 EPC: 21153724.6 / 3 845 116; 18704735.2 / 3 547 893
(73) Proprietor: Boston Scientific Scimed, Inc., Maple Grove, MN 55113 (US)
(72) Inventor: CALABRESE, Andrew, Lancaster, MA 01523 (US); BARBATO, Louis J., Franklin, MA 02038 (US); GAFFNEY, Brian, Rutland, MA 01543 (US); WOOD, Mark D., Sterling, MA 01564 (US)
(74) Representative: Vossius & Partner Patentanwälte Rechtsanwälte mbB

(56) References cited:
- JP-A- 2010 012 079
- JP-A- H07 194 524
- US-A1- 2007 293 719

## Description

.

### Technical Field

The present disclosure relates generally to an endoscope having multiple viewing directions and an adjustable elevator to provide multiple working directions.

### Background

Endoscopic retrograde cholangiopancreatography (ERCP) is a procedure used to diagnose and treat patients with pancreatic-biliary disorders. ERCPs are commonly completed using a side viewing endoscope with an elevator mechanism. The layout of a side viewing scope and elevator feature allow the physician to cannulate the duodenal papilla and access a target site with tools for diagnostic and therapeutic ERCP procedures.

Forward viewing endoscopes are occasionally used in patients with altered anatomy, but since such endoscopes do not feature an elevator or a side viewing image sensor, the physician's diagnostic and therapeutic capabilities may be limited compared with using a side viewing endoscope having an elevator and a side viewing image sensor. In addition, the current commercially available forward viewing scopes may be longer than a side viewing scope and may require different cannulation methods and different tools to be used. The commercially available forward viewing endoscopes may succeed in certain patients with altered anatomy; however, their use may require changes to the physician's procedure and additional effort when compared to performing an ERCP with a side viewing scope.

JP 2010 012079 A relates to an endoscope which includes a circuit board for side viewing which is arranged in parallel with an axial line of the distal end part and allows an imaging element for side viewing mounted on a circuit formation surface to receive light from a direction crossing with the axial line, and a circuit board for straight viewing which is arranged on the opposite surface of the circuit formation surface of the circuit board for side viewing in parallel so as to face the opposite surface of the circuit formation surface and allows an imaging element for straight viewing mounted on the circuit formation surface via a deflecting means to receive light in a direction along the axial line.

JP H07 194524 A relates to a device for raising a treatment tool of an endoscope. A channel tube for passing the treatment tool is disposed at an end of a tip portion. A tip pipe is secured to a tip rising portion, and a wire is connected to the tip pipe. The wire is passed through a guide hole open on an inclined surface of the tip portion.

US 2007/293719 relates to an endoscopic instrument assembly, comprising: an endoscope having a handle portion and an elongate shaft portion, the shaft portion having a working channel defined therein for receiving a first medical device disposed in the working channel; and a locking member disposed adjacent a distal end region of the working channel, the locking member being configured to reversibly secure the position of the first medical device when received within the working channel.

### SUMMARY

The invention is defined in the appended claims. Aspects, embodiments and examples disclosed herein which do not fall within the scope of the appended claims do not form part of the invention and are merely provided for illustrative purposes.

### BRIEF DESCRIPTION OF THE DRAWINGS

The accompanying drawings, which are incorporated in and constitute a part of this specification, illustrate examples of the present disclosure and together with the description, serve to explain the principles of the disclosure.
FIG. 1 illustrates a perspective view of the distal end of an endoscope with a tubular member in a straight configuration to create a front-exit working channel, according to one example.
FIG. 2 illustrates a perspective view of the distal end of the endoscope of FIG. 1, with the tubular member in a bent configuration to create a side-exit working channel, according to one example.
FIG. 3 illustrates a perspective view of the distal end of the endoscope of FIG. 1, with a medical device in the tubular member in a locked position, according to one example.
FIG. 4 illustrates a distal-end view of the endoscope of FIG. 1, according to one example.
FIG. 5 illustrates a top view of the tip of the endoscope of FIG. 2, according to one example.

### DETAILED DESCRIPTION

The present disclosure is drawn to an endoscope having multiple viewing directions. To achieve imaging in multiple directions, the endoscope may have more than one image sensor. According to the claimed invention, the endoscope has an elevator (e.g., a tubular member) with an adjustable distal portion, allowing the endoscope to have both a front-exit working channel and a side-exit working channel.

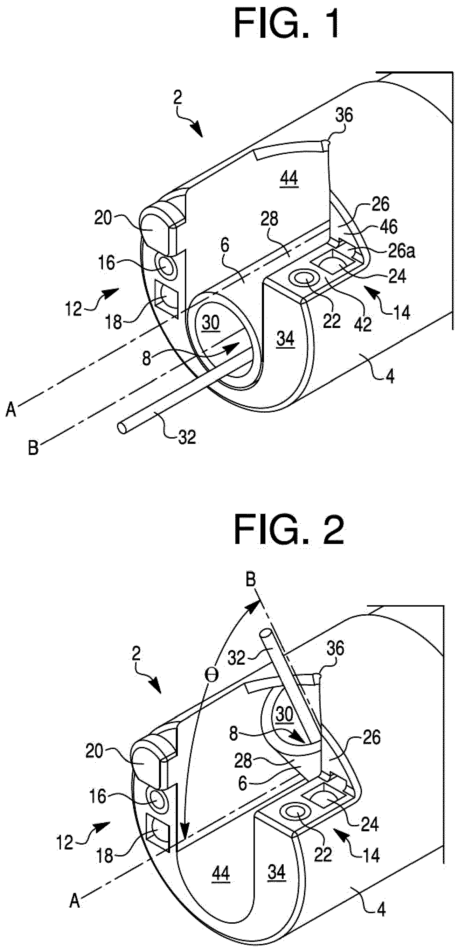

Referring to FIG. 1, an endoscope 2 according to the invention includes a shaft having a distal tip 4. The shaft may be elongate, flexible and tubular. Distal tip 4 may bend left, right, up, and down relative to a proximal portion of the tubular shaft. According to the invention as defined in independent claim 1, endoscope 2 includes an elevator or tubular member 6 defining a lumen 8. The endoscope may include a forward viewing system 12, and a lateral viewing system 14. The tubular shaft (or distal tip 4) of endoscope 2 may define a longitudinal axis A extending in a proximal-distal direction through a center of distal tip 4. In other examples, longitudinal axis A may extend in a proximal-distal direction through distal tip 4 but may be off-center. The forward viewing system 12 may include an image sensor 16, a light source 18, and a lens wash 20. Similarly, the lateral viewing system 14 may include an image sensor 22, a light source 24, and a lens wash 26. Endoscope 2 may be any device used to visually examine the anatomy of a patient and may be used for any suitable procedure. For example, endoscope 2 may be used to examine a patient's gastro-intestinal system, vascular system, respiratory system, or urinary system.

Tubular member 6 may include an elongated tube that extends from a proximal end of the endoscope 2 to a distal end of endoscope 2. Tubular member 6 may include any appropriate biocompatible material, such as polymers, urethane, fluorine-polymer blend, or metals (e.g., stainless steel). Tubular member 6 includes a distal portion 28, shown in the figures, that is bendable relative to a proximal portion (not shown) of tubular member 6. To facilitate bending in a desired region between the proximal portion of tubular member 6 and distal portion 28, tubular member 6 may include a coiled tube portion; a laser-cut pattern that creates a weakened, easily bendable region; a hinge; or any other suitable feature that allows the two portions to bend relative to each other.

Tubular member 6 has a lumen 8. In one example, lumen 8 may have a circular cross-section. In other examples, the cross-section of lumen 8 may be elliptical, polygonal, irregular, or any other suitable shape. Lumen 8 has a distal-end opening 30 at the distal end of distal portion 28. Lumen 8 (or distal portion 28 of tubular member 6) defines a longitudinal axis B extending through a center of distal portion 28 from a proximal end (e.g., adjacent to the bendable region of tubular member 6) to distal-end opening 30. In other examples, longitudinal axis B may extend in a proximal-distal direction through distal portion 28 but may be off-center. Opening 30 provides an outlet for a medical device 32, such as a guide wire or other therapeutic or diagnostic device. To position the medical device 32 in lumen 8, as shown in FIG. 1, a distal end of medical device 32 is inserted into the proximal end of tubular member 6, through lumen 8, and out of distal-end opening 30.

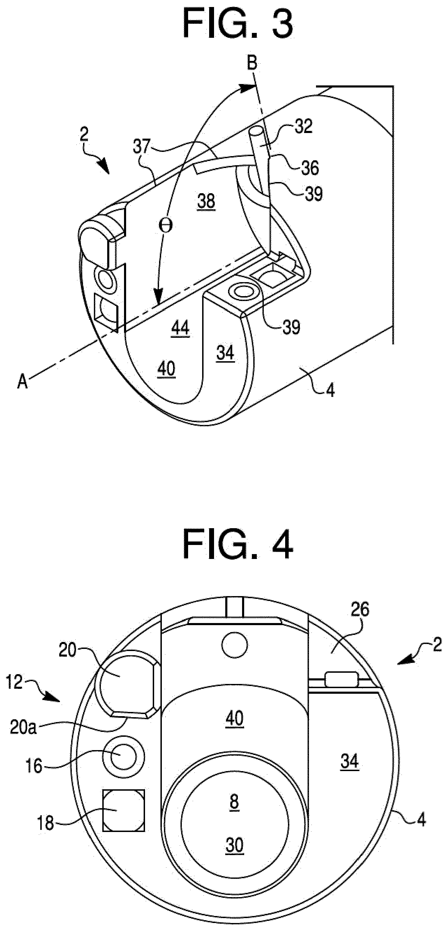

Distal portion 28 of tubular member 6 is movable between a first configuration, shown in FIG. 1, and a second configuration, shown in FIG. 2. FIG. 3 illustrates the tubular member in a third configuration. It should be understood that distal portion 28 is further movable between the first and third configurations and the second and third configurations. In the first configuration of FIG. 1, distal-end opening 30 may be parallel to a distal-end wall 34 of distal tip 4. Furthermore, longitudinal axis B of lumen 8 may be parallel to longitudinal axis A of the tubular shaft (or distal tip 4) of endoscope 2. Accordingly, in the first configuration, tubular member 6 may define a working channel (lumen 8) of endoscope 2 having a front exit. The front exit may be proximate to or in a same plane as distal-end wall 34. When tubular member 6 is in the first configuration, forward viewing system 12 may be used to visualize tools, such as medical device 32, extending from tubular member 6.

When tubular member 6 is in the second configuration, shown in FIG. 2, distal-end opening 30 may form an angle with distal-end wall 34 of distal tip 4. Similarly, longitudinal axis B of distal portion 28 of tubular member 6 may form an angle θ with longitudinal axis A of the tubular shaft (or distal tip 4) of endoscope 2. Accordingly, in the second configuration, tubular member 6 may define a working channel (lumen 8) of endoscope 2 having a side or lateral exit. When tubular member 6 is in the second configuration, lateral viewing system 14 may be used to visualize tools, such as medical device 32, extending from tubular member 6.

FIG. 3 illustrates tubular member 6 in a third configuration, which may be referred to as a locked configuration. In the third configuration, movement of medical device 32 relative to tubular member 6 may be inhibited or prevented. According to the invention, in the third configuration the medical device is locked relative to a wall of the shaft. In one example, the third configuration may be a position of tubular member 6 when distal portion 28 of tubular member 6 is in its maximally bent position (with a medical device 32 extending from distal-end opening 30) relative to the longitudinal axis of distal tip 4 of endoscope 2. In other words, in the third configuration, angle θ may be greater than angle θ when tubular member 6 is in the first or second configurations. Distal tip 4 may include a notch or indentation 36 sized to receive and secure medical device 32 relative to distal tip 4 of the tubular shaft. Indentation 36 may be distal-facing. When tubular member 6 moves to the third configuration, medical device 32 may be pressed between a wall of distal portion 28 and indentation 36 and may form a press-fit attachment with distal tip 4 via indentation 36. In the third configuration of tubular member 6, as will be described further below, medical device 32 may be held securely while a catheter or other medical device is advanced over medical device 32 within lumen 8. Advancement of a medical device over device 32 may occur, however, in any of the first, second, or third configurations.

Referring to FIGs. 3, 4, and 5, the walls of distal tip 4 of the tubular shaft of endoscope 2 include a lateral opening 38 and a distal opening 40 (in distal-end wall 34) to allow tools exiting distal-end opening 30 of tubular member 6 to access an exterior of endoscope 2. Openings 38 and 40 combine to form a single opening at distal tip 4 that is both distal facing and side facing. Lateral opening 38 may extend proximally from distal-end wall 34. From distal-end wall 34, a first side 37 and a second side 39 of lateral opening 38 may extend parallel to each other for a portion of the length (in a proximal-distal direction) of lateral opening 38. The two sides 37, 39 may then move proximally and towards each other towards indentation 36. Indentation 36 may be a part of lateral opening 38 and may be defined on a proximal side by a curved portion of the wall of distal tip 4 extending between first and second sides 37, 39.

Distal opening 40 may be the distal end of a lumen 44 that extends in a longitudinal direction through distal tip 4 of the tubular shaft of endoscope 2. Referring to the view of FIG. 4, distal opening 40 may extend from a top edge of distal tip 4, where it connects and is continuous with lateral opening 38. From lateral opening 38, distal opening 40 may extend towards the opposite wall of distal tip 4 (towards the bottom of the page when viewing FIG. 4). At its bottom edge (in the view of FIG. 4), distal opening 40 may be curved so that the walls of distal-end wall 34 around distal opening 40 form a U-shape. The U-shape of distal opening 40 may allow distal portion 28 of tubular member 6 to extend to the fully straight, first configuration shown in FIGs. 1 and 4. In one example, the curvature of distal opening 40 at the bottom may match the curvature of the outer surface of tubular member 6.

When tubular member 6 is moved from the first configuration to the second or third configurations, medical device 32 may continually have access to an exterior of endoscope 2 via distal opening 40 and lateral opening 38. For example, in the first configuration shown in FIG. 1, medical device 32 may access an exterior of endoscope 2 through distal opening 40. As tubular member 6 approaches the second configuration shown in FIG. 2, medical device 32 may pass through lateral opening 38 to extend from lumen 8 to the exterior of endoscope 2. Accordingly, distal opening 40 and lateral opening 38 provide a pathway so that tools extending from the distal-end opening 30 of lumen 8 can access the exterior of endoscope 2. Described another way, when tubular member 6 is in the first configuration, longitudinal axis B of tubular member 6 may extend through distal opening 40. However, in the second configuration of tubular member 6, longitudinal axis B may extend through lateral opening 38. In the third configuration of tubular member 6, longitudinal axis B may extend through a wall of distal tip 4 if longitudinal axis B passes through a center of lumen 8 (e.g., as shown in FIG. 3). However, if longitudinal axis B is off-center, it also may extend through lateral opening 38 when tubular member 6 is in the third configuration.

Tubular member 6 may be moved through its range of motion (e.g., between first, second, and third configurations) by any mechanism known in the art. In one example, a wire (not shown) may be secured to the exterior of distal portion 28 (e.g., by a ring around tubular member 6, by adhesive, by solder, etc.) and may travel proximally towards the proximal end of tubular member 6. A user may pull the wire proximally to move distal portion 28 from the first configuration towards the second or third configurations. In other words, proximal movement of the wire may cause distal portion 28 to bend further relative to distal tip 4 of endoscope 2 (e.g., may cause angle θ to increase). For convenience, the bending motion of distal portion 28 that increases angle θ will be referred to herein as "proximal" movement of distal portion 28. Conversely, the user may push a wire or other mechanism distally to move distal portion 28 from the second or third configurations towards the first configuration. In other words, distal movement of the wire may cause distal portion 28 to bend less relative to distal tip 4 of endoscope 2 (e.g., may cause angle θ to decrease) and move towards a position in which longitudinal axis B is parallel to longitudinal axis A. For convenience, this straightening motion of distal portion 28 that deceases angle θ will be referred to herein as "distal" movement of distal portion 28.

In one example, distal portion 28 of tubular member 6 may have a range of motion of about 85°. As used herein, "about" means plus or minus 5%, inclusive. Depending on the example, the range of motion may be defined as the angle θ between the first and second configurations (e.g., between FIGs. 1 and 2) or the angle θ between the first and third configurations (e.g., between FIGs. 1 and 3). When the range of motion is the angle θ between the first and second configurations, the range of motion may include any position of distal portion 28 that allows medical device 32 to freely move while protruding from distal-end opening 30 of distal portion 28. When the range of motion is the angle θ between the first and third configurations, the range of motion may include any position of distal portion 28 that allows medical device 32 to protrude from distal-end opening of distal portion 28, whether the medical device 32 is freely movable or locked.

In other examples, distal portion 28 may have a range of motion of about 70°, about 75°, about 80°, or about 90°. In yet other examples, the range of motion of distal portion 28 may be at least 80°, at least 85°, at least 90°, at least 95°, less than 95°, less than 90°, less than 85°, or less than 80°. To increase the range of motion from that shown in the figures, lateral opening 38 of distal tip 4 may be extended proximally to allow distal portion 28 and any medical device 32 within its lumen to bend further in a proximal direction. Alternatively, tubular member 6 may translate or slide distally within lumen 44 of the tubular shaft, sufficiently to permit distal portion 28 to have the desired range of motion. To decrease the range of motion, lateral opening 38 of distal tip 4 may be shortened (e.g., indentation 36 may be moved distally) to prevent distal portion 28 and any medical device 32 within its lumen to bend as far in a proximal direction. Alternatively, tubular member 6 may translate or slide proximally within lumen 44 of the tubular shaft, sufficiently to reduce the range of motion of distal portion 28.

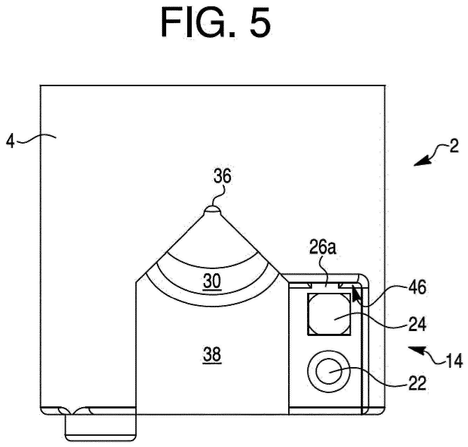

FIG. 4 illustrates a distal-end view of endoscope 2 showing the forward viewing system 12, and FIG. 5 illustrates a top view of distal tip 4 showing lateral viewing system 14. Image sensors 16, 22 may be any sensor that detects light and conveys information to allow for remote viewing of an image, such as CMOS or CCD sensors. Light sources 18, 24 may emit any type of light to illuminate the area proximate to their associated image sensors 16, 22. The lens washes 20, 26 may provide an outlet for water, saline, or other fluid used to clean the respective image sensors 16, 22 and/or light sources 18, 24. Lens wash 20 may include an exit nozzle 20a for fluid to exit lens wash 20. Similarly, lens wash 26 may include an exit nozzle 26a for fluid to exit lens wash 26.

Referring to the distal-end view of FIG. 4, the components of forward viewing system 12 may be positioned on distal-end wall 34 of distal tip 4/tubular shaft/endoscope 2. Distal-end wall 34 may be perpendicular to longitudinal axis A. Image sensor 16 may be positioned towards the left side of distal opening 40. Light source 18 may be positioned below image sensor 16 (in the view of FIG. 4) and may be associated with image sensor 16 by being positioned to illuminate an area proximate to image sensor 16. Lens wash 20 may be positioned above image sensor 16 (in the view of FIG. 4) and may be configured to release fluid to clean image sensor 16 and/or light source 18. However, image sensor 16, light source 18, and lens wash 20 may be positioned in any other order or configuration relative to each other. For example, two or more of the components may be positioned next to each other (side-by-side in the view of FIG. 4). Alternatively, one or more of image sensor 16, light source 18, and lens wash 20 may be positioned on the opposite side of distal opening 40 (e.g., on the portion of distal-end wall 34 to the right of distal opening 40 in the view of FIG. 4, closer to viewing system 14). In an alternative example, one or more of image sensor 16, light source 18, and lens wash 20 may be positioned on a distal-facing wall of the tubular shaft of endoscope 2 that is not at the distal-most end of endoscope 2 (e.g., on a distal wall 46 (see FIG. 1) or another distal-facing wall).

Referring to the top view of FIG. 5, image sensor 22 may be positioned proximate to (and proximal to) distal-end wall 34, and light source 24 may be positioned proximal to image sensor 22. Image sensor 22 and light source 24 may be positioned on a lateral wall 42 of distal tip 4/tubular shaft/endoscope 2. Light source 24 may be associated with image sensor 22 by being positioned to illuminate an area proximate to image sensor 22. Lateral wall 42 may be any lateral-facing wall and may be curved or flat. Referring to FIGs. 1-3, in one example, lateral wall 42 may be a flat wall extending proximally from distal-end wall 34. In another example, lateral wall 42 may be a curved outer surface of distal tip 4. Referring back to FIG. 5, lens wash 26 may be positioned proximal to light source 24 on distal wall 46 and may include an outlet through which saline or another fluid may be released along lateral wall 42 to clean image sensor 22 and/or light source 24. Image sensor 22, light source 24, and lens wash 26 may be positioned in any other order or configuration relative to each other. Alternatively, one or more of these components may be positioned on the opposite side of lateral opening 38 (e.g., on the left of lateral opening 38 in the view of FIG. 5).

Image sensor 16 on distal-end wall 34 may face a distal direction. Accordingly, when tubular member 28 is in the first configuration and when it is moving proximally towards the second configuration, image sensor 16 may be used to view medical device 32 and any other tools extending from distal-end opening 30. In contrast, image sensor 22 in lateral wall 42 may face a lateral direction. Accordingly, when tubular member 28 is approaching the second configuration or when it is in the second or third configuration, image sensor 22 may be used to view medical device 32 and any other tools extending from distal-end opening 30.

In one example, distal-end wall 34 (or any other distal-facing wall that includes image sensor 16) and lateral wall 42 (or any other lateral-facing wall that includes image sensor 22) may be perpendicular to each other. The angles described herein between the two walls may correspond to the angles between the viewing directions of image sensors 16, 22. In another example, distal-end wall 34 and lateral wall 42 (and the corresponding viewing directions of image sensors 16, 22) may deviate from 90° and may be positioned about 90° relative to each other. In other examples, the two walls may be positioned about 85°, about 80°, or about 75° relative to each other. In yet another example, the two walls may be positioned at least 90° or less than 90° relative to each other.

In one example, the viewing directions of image sensors 16, 22 can be adjusted relative to their respective walls. For example, the viewing direction of image sensor 16 may be adjusted to pivot along one or more axes relative to its default position within distal-end wall 34, with the default position being a position facing the same direction as distal-end wall 34. Similarly, the viewing direction of image sensor 22 may be adjusted to pivot along one or more axes relative to its default position within lateral wall 42, with the default position being a position facing the same direction as lateral wall 42.

In an exemplary procedure using endoscope 2, distal tip 4 may be inserted through a patient's mouth, esophagus, stomach, and duodenum during a first portion of the procedure. Forward viewing system 12 may be used during the first portion of the procedure to help the user navigate through the patient's anatomy. While navigating to the duodenum, tubular member 6 may be in a first configuration to allow medical device 32 (e.g., a guide wire) or other tools to move distally past the distal-end wall 34 of distal tip 4. For example, a guide wire may be inserted from the stomach and through the pylorus to help navigate the distal tip 4 from the stomach into the duodenum.

Near the duodenal papilla, the user may use lateral viewing system 14 to help locate the duodenal papilla. Using guidance from lateral viewing system 14, medical device 32 (e.g., a guide wire) may be inserted through the duodenal papilla and into the patient's pancreatic duct. To aid in inserting medical device 32 into the duodenal papilla, tubular member 6 may be moved to the second configuration to allow medical device 32 to exit the lateral side of endoscope 2. Endoscope 2 and its distal tip 4 may remain in a substantially straight configuration within the duodenum while medical device 32 extends out of distal-end opening 30 from the lateral side of distal tip 4. Once medical device 32 is inserted into the pancreatic duct, tubular member 6 may be moved to the third configuration to lock medical device 32 relative to distal tip 4 of the tubular shaft. In one example, both viewing systems 12, 14 may be used simultaneously during any portion of a procedure.

A cannula (not shown) then may be inserted through lumen 8 of tubular member 6 over medical device 32. Once the cannula reaches distal tip 4, tubular member 6 may be moved proximally to the second configuration (or past the second configuration towards the first configuration) to unlock medical device 32. The cannula may then be passed out of distal-end opening 30 and into the pancreatic duct over medical device 32 to cannulate the pancreatic duct. A contrast agent may be injected into the pancreatic duct, and fluoroscopy images may be taken to study the bile ducts, the gallbladder, and the pancreatic duct. If desired, other tools may be inserted through the tubular member 6 and/or cannula into the pancreatic duct to treat pancreatic or biliary conditions.

## Claims

1. An endoscope (2), comprising
a shaft defining a longitudinal axis (A) and having a distal tip (4),
an elevator (6) having a lumen (8), wherein the elevator (6) includes a distal portion (28) that is bendable relative to a proximal portion of the elevator (6), the lumen (8) having a distal-end opening (30), and the distal portion (28) of the elevator (6) defining a longitudinal axis (B) through a center of the distal portion (28), the distal portion (28) of the elevator (6) being movable between a first configuration, a second configuration, and a third configuration, and
a medical device (32) positioned within the elevator (6),
wherein walls of the distal tip (4) include a lateral opening (38) and a distal opening (40) to allow the medical device (32) exiting the distal-end opening (30) of the elevator (6) to access an exterior of the endoscope (2),
wherein the lateral opening (38) and the distal opening (40) combine to form a single opening at the distal tip (4) that is both distal facing and side facing,
wherein, when the distal portion (28) of the elevator (6) is in the third configuration, the medical device (32) is locked relative to a wall of the shaft.

2. The endoscope of claim 1, wherein the distal opening (40) and the lateral opening (38) provide a pathway so that the medical device (32) extending from the distal-end opening (30) of the lumen (8) can access the exterior of the endoscope (2).

3. The endoscope of claim 1 or 2, wherein when the distal portion (28) of the elevator (6) is moved from the first configuration to the second configuration, the medical device (32) continually has access to the exterior of the endoscope (2) via the distal opening (40) and the lateral opening (38).

4. The endoscope of any of the preceding claims, wherein when the distal portion (28) of the elevator (6) is in the first configuration, the longitudinal axis (B) of the distal portion (28) of the elevator (6) extends through the distal opening (40), and wherein when the distal portion (28) of the elevator (6) is in the second configuration, the longitudinal axis (B) extends through the lateral opening (38).

5. The endoscope of any of the preceding claims, wherein when the distal portion (28) of the elevator (6) is in the first configuration, the distal-end opening (30) is parallel to a distal-end wall (34) of the distal tip (4).

6. The endoscope of claim 5, wherein the distal-end wall (34) is perpendicular to the longitudinal axis (A) of the shaft.

7. The endoscope of claim 5 or 6, wherein when the distal portion (28) of the elevator (6) is in the first configuration, the elevator (6) defines a working channel of the endoscope (2) having a front exit that is proximate to or in a same plane as the distal-end wall (34).

8. The endoscope of any of the preceding claims, wherein the distal tip (4) of the shaft includes an indentation (36) sized to receive and secure the medical device (32) relative to the distal tip (4).

9. The endoscope of any of the preceding claims,
wherein when the distal portion (28) of the elevator (6) is in the third configuration, movement of the medical device (32) relative to the elevator (6) is inhibited or prevented.

10. The endoscope of claim 9, comprising the features of claim 8, wherein when the distal portion (28) of the elevator (6) moves to the third configuration, the medical device (32) is pressed between a wall of the distal portion (28) and the indentation (36) and forms a press-fit attachment with the distal tip (4) via the indentation (36).

11. The endoscope of any of the preceding claims, wherein when the distal portion (28) of the elevator (6) is in the second configuration, the longitudinal axis (B) of the distal portion (28) of the elevator (6) forms an angle (θ) with the longitudinal axis (A) of the shaft.

12. The endoscope of claim 11, wherein when the distal portion (28) of the elevator (6) is in the third configuration, the longitudinal axis (B) of the distal portion (28) of the elevator (6) forms a further angle (θ) with the longitudinal axis (A) of the shaft that is greater than the angle (θ) when the distal portion (28) of the elevator (6) is in the second configuration.

13. The endoscope of any of the preceding claims, further comprising a wire secured to an exterior of the distal portion (28) of the elevator (6) and travelling proximally towards a proximal end of the elevator (6), configured to be pulled proximally by a user to move the distal portion (28) of the elevator (6) from the first configuration towards the second configuration or the third configuration.

14. The endoscope of any of the preceding claims, wherein the endoscope further comprises a first image sensor (16) facing a distal direction, and a second image sensor (22) facing a lateral direction.

## Patentansprüche

1. Endoskop (2), das aufweist:
einen Schaft, der eine Längsachse (A) definiert und eine distale Spitze (4) hat, einen Elevator (6) mit einem Lumen (8), wobei der Elevator (6) einen distalen Abschnitt (28) aufweist, der relativ zu einem proximalen Abschnitt des Elevators (6) biegbar ist, das Lumen (8) eine am distalen Ende liegende Öffnung (30) hat und der distale Abschnitt (28) des Elevators (6) eine Längsachse (B) durch eine Mitte des distalen Abschnitts (28) definiert, wobei der distale Abschnitt (28) des Elevators (6) zwischen einer ersten Konfiguration, einer zweiten Konfiguration und einer dritten Konfiguration bewegbar ist, und
eine medizinische Vorrichtung (32), die im Elevator (6) positioniert ist,
wobei Wände der distalen Spitze (4) eine laterale Öffnung (38) und eine distale Öffnung (40) aufweisen, damit die medizinische Vorrichtung (32), die aus der am distalen Ende liegenden Öffnung (30) des Elevators (6) austritt, Zugang zu einer Außenseite des Endoskops (2) erhält,
wobei sich die laterale Öffnung (38) und die distale Öffnung (40) zu einer einzigen Öffnung an der distalen Spitze (4) kombinieren, die sowohl distal weist als auch seitlich weist,
wobei in der dritten Konfiguration des distalen Abschnitts (28) des Elevators (6) die medizinische Vorrichtung (32) relativ zu einer Wand des Schafts arretiert ist.

2. Endoskop nach Anspruch 1, wobei die distale Öffnung (40) und die laterale Öffnung (38) einen Durchgang bereitstellen, so dass die sich aus der am distalen Ende liegenden Öffnung (30) des Lumens (8) erstreckende medizinische Vorrichtung (32) Zugang zur Außenseite des Endoskops (2) erhalten kann.

3. Endoskop nach Anspruch 1 oder 2, wobei bei Bewegung des distalen Abschnitts (28) des Elevators (6) aus der ersten Konfiguration in die zweite Konfiguration die medizinische Vorrichtung (32) ständigen Zugang zur Außenseite des Endoskops (2) über die distale Öffnung (40) und die laterale Öffnung (38) erhält.

4. Endoskop nach einem der vorstehenden Ansprüche, wobei sich in der ersten Konfiguration des distalen Abschnitts (28) des Elevators (6) die Längsachse (B) des distalen Abschnitts (28) des Elevators (6) durch die distale Öffnung (40) erstreckt und wobei sich in der zweiten Konfiguration des distalen Abschnitts (28) des Elevators (6) die Längsachse (B) durch die laterale Öffnung (38) erstreckt.

5. Endoskop nach einem der vorstehenden Ansprüche, wobei in der ersten Konfiguration des distalen Abschnitts (28) des Elevators (6) die am distalen Ende liegende Öffnung (30) parallel zu einer am distalen Ende liegenden Wand (34) der distalen Spitze (4) ist.

6. Endoskop nach Anspruch 5, wobei die am distalen Ende liegende Wand (34) senkrecht zur Längsachse (A) des Schafts ist.

7. Endoskop nach Anspruch 5 oder 6, wobei in der ersten Konfiguration des distalen Abschnitts (28) des Elevators (6) der Elevator (6) einen Arbeitskanal des Endoskops (2) definiert, der einen vorderen Ausgang hat, der nahe der am distalen Ende liegenden Wand (34) oder in derselben Ebene wie diese liegt.

8. Endoskop nach einem der vorstehenden Ansprüche, wobei die distale Spitze (4) des Schafts eine Einbuchtung (36) aufweist, die so bemessen ist, dass sie die medizinische Vorrichtung (32) relativ zur distalen Spitze (4) aufnimmt und befestigt.

9. Endoskop nach einem der vorstehenden Ansprüche,
wobei in der dritten Konfiguration des distalen Abschnitts (28) des Elevators (6) eine Bewegung der medizinischen Vorrichtung (32) relativ zum Elevator (6) gehemmt oder verhindert ist.

10. Endoskop nach Anspruch 9 mit den Merkmalen von Anspruch 8, wobei bei Bewegung des distalen Abschnitts (28) des Elevators (6) in die dritte Konfiguration die medizinische Vorrichtung (32) zwischen einer Wand des distalen Abschnitts (28) und der Einbuchtung (36) gedrückt wird und eine Presspassung mit der distalen Spitze (4) über die Einbuchtung (36) bildet.

11. Endoskop nach einem der vorstehenden Ansprüche, wobei in der zweiten Konfiguration des distalen Abschnitts (28) des Elevators (6) die Längsachse (B) des distalen Abschnitts (28) des Elevators (6) einen Winkel (θ) mit der Längsachse (A) des Schafts bildet.

12. Endoskop nach Anspruch 11, wobei in der dritten Konfiguration des distalen Abschnitts (28) des Elevators (6) die Längsachse (B) des distalen Abschnitts (28) des Elevators (6) einen weiteren Winkel (θ) mit der Längsachse (A) des Schafts bildet, der größer ist als der Winkel (θ), wenn sich der distale Abschnitt (28) des Elevators (6) in der zweiten Konfiguration befindet.

13. Endoskop nach einem der vorstehenden Ansprüche, ferner mit einem Draht, der an einer Außenseite des distalen Abschnitts (28) des Elevators (6) befestigt ist und hin zu einem proximalen Ende des Elevators (6) proximal verläuft und so konfiguriert ist, dass er von einem Benutzer proximal gezogen wird, um den distalen Abschnitt (28) des Elevators (6) aus der ersten Konfiguration hin zur zweiten Konfiguration oder dritten Konfiguration zu bewegen.

14. Endoskop nach einem der vorstehenden Ansprüche, wobei das Endoskop ferner aufweist: einen ersten Bildsensor (16), der in eine distale Richtung weist, und einen zweiten Bildsensor (22), der in eine laterale Richtung weist.

## Revendications

1. Endoscope (2) comprenant
une tige définissant un axe longitudinal (A) et ayant une pointe distale (4);
un élévateur (6) ayant une lumière (8), dans lequel l'élévateur (6) inclut une partie distale (28) qui peut fléchir par rapport à une partie proximale de l'élévateur (6), la lumière (8) ayant une ouverture d'extrémité distale (30), et la partie distale (28) de l'élévateur (6) définissant un axe longitudinal (B) par un centre de la partie distale (28), la partie distale (28) de l'élévateur (6) étant mobile entre une première configuration, une seconde configuration et une troisième configuration, et
un dispositif médical (32) positionné à l'intérieur de l'élévateur (6),
dans lequel les parois de la pointe distale (4) incluent une ouverture latérale (38) et une ouverture distale (40) pour permettre au dispositif médical (32) sortant de l'ouverture d'extrémité distale (30) de l'élévateur (6) d'accéder à un extérieur de l'endoscope (2),
dans lequel l'ouverture latérale (38) et l'ouverture distale (40) se combinent pour former une seule ouverture au niveau de la pointe distale (4) qui à la fois fait face au côté distal et fait face au côté latéral,
dans lequel, lorsque la partie distale (28) de l'élévateur (6) est dans la troisième configuration, le dispositif médical (32) est verrouillé par rapport à une paroi de la tige.

2. Endoscope selon la revendication 1, dans lequel l'ouverture distale (40) et l'ouverture latérale (38) forment un passage de sorte que le dispositif médical (32) s'étendant depuis l'ouverture d'extrémité distale (30) de la lumière (8) peut accéder à l'extérieur de l'endoscope (2).

3. Endoscope selon la revendication 1 ou 2, dans lequel, lorsque la partie distale (28) de l'élévateur (6) est déplacée de la première configuration à la seconde configuration, le dispositif médical (32) a un accès de manière continue à l'extérieur de l'endoscope (2) par l'intermédiaire de l'ouverture distale (40) et de l'ouverture latérale (38).

4. Endoscope selon l'une quelconque des revendications précédentes, dans lequel, lorsque la partie distale (28) de l'élévateur (6) est dans la première configuration, l'axe longitudinal (B) de la partie distale (28) de l'élévateur (6) s'étend à travers l'ouverture distale (40), et dans lequel, lorsque la partie distale (28) de l'élévateur (6) est dans la seconde configuration, l'axe longitudinal (B) s'étend à travers l'ouverture latérale (38).

5. Endoscope selon l'une quelconque des revendications précédentes, dans lequel, lorsque la partie distale (28) de l'élévateur (6) est dans la première configuration, l'ouverture d'extrémité distale (30) est parallèle à une paroi d'extrémité distale (34) de la pointe distale (4).

6. Endoscope selon la revendication 5, dans lequel la paroi d'extrémité distale (34) est perpendiculaire à l'axe longitudinal (A) de la tige.

7. Endoscope selon la revendication 5 ou 6, dans lequel, lorsque la partie distale (28) de l'élévateur (6) est dans la première configuration, l'élévateur (6) définit un canal de travail de l'endoscope (2) ayant une sortie avant qui est proche de ou dans un même plan que la paroi d'extrémité distale (34).

8. Endoscope selon l'une quelconque des revendications précédentes, dans lequel la pointe distale (4) de la tige inclut une indentation (36) dimensionnée pour recevoir et fixer le dispositif médical (32) par rapport à la pointe distale (4).

9. Endoscope selon l'une quelconque des revendications précédentes, dans lequel, lorsque la partie distale (28) de l'élévateur (6) est dans la troisième configuration, le mouvement du dispositif médical (32) par rapport à l'élévateur (6) est entravé ou empêché.

10. Endoscope selon la revendication 9, comprenant les caractéristiques de la revendication 8, dans lequel, lorsque la partie distale (28) de l'élévateur (6) se déplace jusqu'à la troisième configuration, le dispositif médical (32) est pressé entre une paroi de la partie distale (28) et l'indentation (36) et forme une fixation par ajustage serré avec la pointe distale (4) par l'intermédiaire de l'indentation (36).

11. Endoscope selon l'une quelconque des revendications précédentes, dans lequel, lorsque la partie distale (28) de l'élévateur (6) est dans la seconde configuration, l'axe longitudinal (B) de la partie distale (28) de l'élévateur (6) forme un angle (θ) avec l'axe longitudinal (A) de la tige.

12. Endoscope selon la revendication 11, dans lequel, lorsque la partie distale (28) de l'élévateur (6) est dans la troisième configuration, l'axe longitudinal (B) de la partie distale (28) de l'élévateur (6) forme, avec l'axe longitudinal (A) de la tige, un autre angle (θ) qui est supérieur à l'angle (θ) lorsque la partie distale (28) de l'élévateur (6) est dans la seconde configuration.

13. Endoscope selon l'une quelconque des revendications précédentes, comprenant en outre un fil fixé à un extérieur de la partie distale (28) de l'élévateur (6) et s'étendant de manière proximale vers une extrémité proximale de l'élévateur (6), configuré pour être tiré de manière proximale par un utilisateur pour déplacer la partie distale (28) de l'élévateur (6) de la première configuration vers la seconde configuration ou la troisième configuration.

14. Endoscope selon l'une quelconque des revendications précédentes, dans lequel l'endoscope comprend en outre un premier capteur d'image (16) faisant face à une direction distale et un second capteur d'image (22) faisant face à une direction latérale.
